# EUROPEAN PATENT APPLICATION

(11) **EP 1 905 467 A1**
(43) Date of publication of application: **02.04.2008**
(21) Application number: 06254966.2
(22) Date of filing: 26.09.2006
(51) Int. Cl.: A61M 5/32

(54) **Needle safety device**

(71) Applicant: The Royal College of Art, London SW7 2EU (GB); Korn, Michael, London SW7 2EU (GB)
(72) Inventor: The designation of the inventor has not yet been filed
(74) Representative: Hedley, Nicholas James Matthew

(57) **Abstract**

A device is described that is configured to grip the sheath (16) of a medical needle so that the needle can be retracted from it with one hand and to hold the sheath while the needle is in use, which device comprises at least two elements (20,22) that are resiliently connected together, e.g. by a hinge (24) and at least partially define at least one opening (26,28) into which the sheath can be pushed, said at least two elements and at least one opening acting as a valve that allows the sheath to be pushed into the at least one opening but grips the sheath if the sheath is pulled out of the opening, wherein the resilient connection between the elements is configured to hold a sheath against rotating within the opening so that a syringe can be twisted off the needle held in the device.

## Description

### Technical Field

The present invention relates to a device for assisting in the removal and replacement of the sheath that covers a medical needle, e.g. a hypodermic needle. The term "medical needle" and similar expressions used in the present specification is intended to cover any needle used in medicine, especially those used with a syringe or tubing, whether for injecting liquids into a human or animal patient or for removing materials from the patient, e.g. a catheter needle, and includes needles used during non-therapeutic application, e.g. in laboratory research.

### Background Art

"Needle stick injury" is a common and dangerous problem for all users of medical needles and describes the accidental jabbing of oneself or others with used, contaminated needles, which can lead to health care workers contracting blood-borne diseases, e.g. HIV infection and hepatitis, from the patients they are treating. Needle stick injury is the second most commonly reported accident in the UK National Health Service, with around 100,000 cases reported annually. The conventional method to make a needle safe after use is to re-sheath the used needle, i.e. push it back into its protective plastic sheath. However, it is common for needlestick accidents to occur while a health worker is actually re-sheathing a contaminated needle as it is easy to miss the sheath and stab oneself and so this procedure has been banned by most UK National Health Service trusts. As such, used needles are carried around hospital wards on open trays and taken to sharps' bins (see below) where they are disposed of. If health workers have a needle stick injury, they must undertake a series of tests to see if they are infected, which is expensive for the health authority. There is also of course a substantial cost associated with treatment of infected health workers, litigation, stress and illness related absenteeism and safety equipment and procedures. These problems cost the UK National Health Service millions of pounds per year. This is a global problem, and is not limited only to health workers but to anyone who uses needles, e.g. diabetes sufferers or their carers.

Currently, when an injection is to be given, an ampoule containing the injectable solution is placed on a small hospital tray together with a medical syringe and needle for delivering the solution; the tray with the solution, syringe and needle is supplied to a nurse or doctor or is prepared by the health workers themselves. The tray is often made of paper-pulp. After use, the tray, the syringe and the needle are all disposed of.

Hospitals generally separate waste into different types, which are disposed of separately. These may be:
1. Non-contaminated (household) waste;
2. Contaminated waste; and
3. Sharp objects, e.g. hypodermic needles.

The cost of dispensing of category 3 (sharp objects) is dramatically higher than that of contaminated waste, which in turn is higher than that of non-contaminated waste. However, it often happens that the waste is not properly segregated and contaminated waste or non-contaminated waste is disposed of in the containers intended for sharp objects. It has been estimated that around 80% of hospital waste placed in the bins for sharp objects should in fact have been placed in bags for contaminated or non-contaminated waste. This leads to the unnecessary expenditure of the cost of disposing of sharp objects. However, the separation of sharp objects from other objects and the re-sheathing of needles often give rise in itself to needle stick injury, which encourages the disposal of non-sharp objects in bins intended for sharp objects.

There exists a need for a device that allows the needle to be re-sheathed without requiring the medical worker to hold the sheath in the process and without requiring the medical worker to place his/her fingers in the vicinity of the point of a needle. Finally, there exists a need to separate a medical needle from a syringe or other liquid injecting or removing conduit while minimising the risk of needle stick in order to allow the needle and sheath to be disposed of in a container for sharp objects, while allowing the remaining medical equipment used to be disposed of through less expensive channels.

### Disclosure of Invention

By exploiting the different fitting mechanisms that exist between, on the one hand, a needle connector, e.g. on a standard syringe, and a needle and, on the other hand, between a needle and a needle-protective sheath, a device has been designed which enables safe, one-handed disposal of medical needles. This involves insertion of the syringe into the needle base to attach the needle to the syringe, retraction of the needle from its sheath, use of the needle, insertion of the now contaminated needle back into its sheath, removal of the syringe from the needle by a twisting mechanism and then separation and disposal of the sharps and non-sharps.

According to the present invention there is provided a device that is configured to grip the sheath of a medical needle so that the needle can be retracted from it and to hold the sheath while the needle is in use, which device comprises at least two elements that are resiliently connected together and at least partially define at least one opening into which the sheath can be pushed. The at least two elements and the at least one opening act as a valve that allows the sheath to be pushed into the opening along a first direction but grips the sheath if the sheath is pulled out of the opening in a second direction that is opposite to the first direction. In other words, the force needed to pull the sheath out of the device in the second direction is greater than the force needed to push the sheath into the device in the first direction. The device, when holding the sheath will also prevent the sheath from twisting, to allow the syringe to be separated from the needle after use by pushing the needle into the sheath held by the device and applying a twisting movement to the syringe to detach it.

By gripping the sheath, the device allows the medical needle to be retracted from the sheath and pushed back into the sheath while keeping the medical worker's hands away from the needle point. This minimises the danger of needle stick injury. The device can be operated by a health worker (or other user) as follows: attaching the sheathed needle to the syringe, holding the syringe to which the needle and sheath are attached, pushing the sheath into the opening and pulling the syringe in the opposite direction, which causes the sheath to be gripped by the device, and allows the needle to be pulled out of the sheath, which is held by the device. After use, the needle is pushed into the sheath that is held by the device and the syringe is detached by a twisting and pulling movement. The sheathed needle can optionally be removed from the device since it is safe to handle in its sheathed condition and disposed of in the appropriate way or, more preferably, the device and the sheathed needle are disposed of, which means that it is not necessary for the sheathed needle to be removed from the device. The device can be held at a location spaced away from the opening, i.e. the user's fingers are located away from the opening, so that the danger of needle stick is reduced.

In a first embodiment, the device includes two elements that are resiliently connected together by a hinge. Each element includes a hole of a size to accommodate the sheath snugly. The two elements are not parallel to each other so that the holes are not directly aligned with each other. When the sheath is pushed into the holes of the two elements, the elements flex with respect to each other about the resilient hinge, thereby bringing the holes closer into alignment and allowing the sheath to be pushed into the holes. When holding the sheath after it has been pushed in and the pushing force released, the resilience of the hinge causes the hinge to flex back, i.e. so that the holes are less in alignment, whereby the sheath is held by the holes. A force exerted trying to remove the sheath is resisted because the engagement between the sheath and the edges of the holes would move the elements apart, which would take the holes further out of alignment. This means that the elements grip the sheath and resist it from being removed from the device when the sheath is held in the device.

When the sheath is held on the needle by friction or a push fit (also called a "pop" or "snap" fit), the retraction of the needle from the sheath can be effected by a simple pulling action or when the sheath is held on the needle by a screw thread, the retraction of the needle from the sheath can effected by a twisting motion.

This embodiment is not limited only to two elements but further elements, each with holes and each resiliently attached to a preceding element can be used. In this way, the sheath can be gripped by three or more elements in the manner described above.

In a further embodiment, the elements may be resiliently connected together and form an opening between them. In this case, the elements are angled with respect to each other so that the sheath can be pushed in one direction, which tends to expand the opening and thereby allows the sheath to pass through. However, because of the angle between the elements, when the sheath is pulled in the opposite direction, the engagement of the sheath with the elements tends to close the opening and resists the sheath being removed from the device. The elements may be integrally formed, e.g. in a single moulding. Conveniently, the elements can form a dome such that the sheath can be pushed through the concave face of the dome while the elements resist the sheath moving in the opposite direction.

Accordingly to a further aspect of the present invention, the device may be incorporated into a holder, e.g. a hospital tray, that is capable of holding a medical needle, a sheath fitted over the needle and a syringe. The device may be separable from the rest of the holder, e.g. by means of a line of weakness or by being temporarily secured to the tray. This allows re-sheathed needle held in the device to be separated from the syringe; the device and the re-sheathed needle can then be separated from the holder and disposed of in a suitable container for sharp material while allowing the holder and the syringe to be disposed of more cheaply.

The device may be integrally formed with the holder, for example if the holder is a tray made from paper-pulp, the two elements of the device in the first embodiment may be two flaps of paper-pulp attached to the rim of the tray. Because the device can be made integrally with the rest of the holder, its inclusion need not increase the cost of the holder significantly.

Similar considerations apply if the holder were made of plastic materials or other disposable or reusable materials. It is possible to make the device out of metal.

According to a further embodiment of the present invention, there is provided a system for preventing needle stick, the system comprising a needle held within a sheath and a flexible wrapper, the inner surface of the wrapper including a first area of adhesive that adheres the sheath to the wrapper and a second area of contact adhesive that is accessible when the wrapper is open and that can be used to adhere the wrapper to a surface to immobilise the wrapper. Using this arrangement, it is possible to open the wrapper to expose both (a) the part of the needle that connects the needle to a syringe and (b) the second area of contact adhesive on the inside of the wrapper so that the wrapper can be adhered by the second area of adhesive to a surface, e.g. the inside of a bowl or tray. A syringe can then be pushed onto the needle within the wrapper with the adhesive in the first and second areas keeping the wrapper and the sheath in a stable position while this is happening. The needle can then be pulled out from the sheath by pulling on the syringe, leaving the sheath within the wrapper. When the needle has been used, it can be pushed into the sheath within the wrapper but there is no need to hold the sheath to do this since the sheath is held still by the first area of adhesive attaching the sheath to the wrapper and the second area of adhesive adhering the wrapper to the tray. Thus there is no need for the health worker to hold the sheath while the needle is being inserted into the sheath, thereby averting the risk of needle stick injury. The syringe can then be twisted and pulled to remover the syringe from the needle. The wrapper and the sheathed needle can then be pulled off the retaining surface and disposed of in a sharps container or, if need be, the surface, wrapper and sheathed needle could be disposed of together. The first and second areas of adhesive can be a single deposit of adhesive that covers both the first and the second areas.

### Brief Description of the Drawings

There will now be described, by way of example only, two embodiments of the device in accordance with the present invention by reference to the accompanying drawings in which:
Figure 1 is a schematic exploded view of a syringe, needle and sheath or sheath of standard design;
Figure 2 is schematic view of the steps for attaching the hypodermic needle to a syringe, unsheathing the needle, re-sheathing the needle, removing the syringe and disposing of the used needle using a device in accordance with a first embodiment of the present invention;
Figure 3 is a sectional view through part of the first embodiment of the present invention both before it holds a syringe sheath (Figure a) and while it is holding a sheath (Figure b); Figure 3(c) is a plan view of the fingers 27;
Figure 4 is a sectional view through part of a second embodiment of the present invention showing the device before holding a syringe sheath (Figure a) and while holding a syringe sheath (Figure b);
Figure 5 is a sectional view through part of a third embodiment of the present invention showing the device before holding a syringe sheath.

### Best Mode for Putting Invention Into Operation

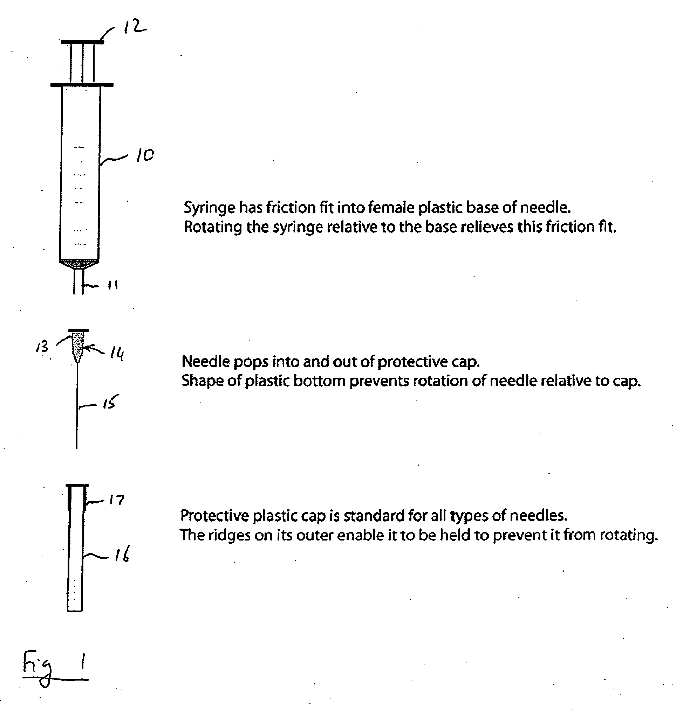

Referring initially to Figure 1, there is shown a syringe 10, needle 14 and sheath 16 of standard design; the same sized sheath is generally used for all needle sizes, although canula needles and vacutainer needles have different sized sheaths. The device of the present invention, if suitably configured, can accommodate a range of different sizes of sheath.

The syringe 10 has a plunger 12 that can be moved downwardly to deliver injectable material through a cylindrical outlet 11. The hypodermic needle 14 includes a hollow needle 15 and a collar 13 that can be pushed onto the syringe outlet 11 and be held there by friction. The frictional engagement can be broken by twisting the syringe 10 relative to the needle 14 and applying a slight pulling away force.

The needle 15 is protected by a plastic sheath or cap 16 that can be friction fitted with a 'pop' fit onto the collar 13 of the needle 14. The engagement is such that the needle cannot rotate readily within the sheath 16. As such, the needle and sheath can be removed as a unit from the syringe 10 by holding the sheath and applying a twisting and pulling motion to the syringe. Ridges 17 are provided on the outside of the sheath to assist in gripping the sheath. In contrast to the twisting motion used to remove the needle/sheath unit as a whole, the sheath 16 can only be removed from the needle by pulling the sheath with a force sufficient to overcome both the friction between the sheath and the needle and also the "pop" fitting. The pop fitting is a combination of at least one projection on either the needle or the sheath that engages with at least one recess in the other. Since the projections and/or recesses are made of deformable material, the pop fitting can be overcome by pulling the sheath with a force necessary to deform the fitting and allow the projection(s) to be removed from the recess(es).

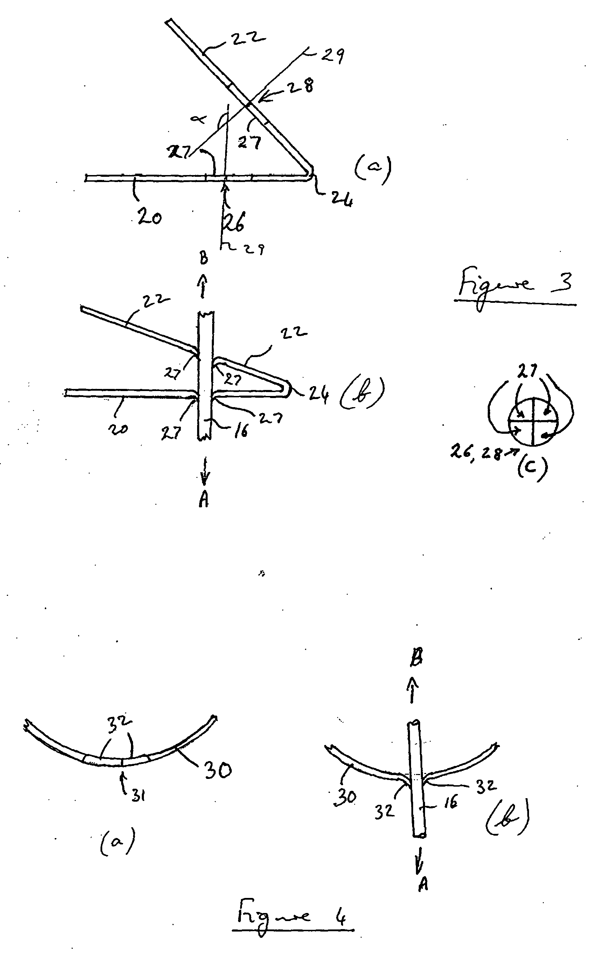

Referring now to Figure 3, there is shown a device in accordance with the present invention, which includes two elements 20, 22 that are connected by a resilient hinge 24. The device can be made simply by folding a sheet along the hinge 24 to make the two elements. This folded hinge may be curved to provide extra stiffness. The device may therefore be made out of any material that is stiff and has resilience, including paper-pulp sheeting, plastic material or metal.

Punched into the elements 20, 22 are two openings 26, 28 formed by cross shapes. Each cross shape forms four resilient fingers 27 in each element that can be pushed out by a sheath 16 of a hypodermic needle, as shown in Figure 3(b), which shows the sheath 16 once it has been pushed in the direction of arrow A through the openings 26, 28; in the process, the movement of the sheath into the openings causes the fingers 27 of the openings to bend downwardly in the direction of arrow A. The bent fingers 27 prevents the sheath being pulled in the direction B (opposite to the direction of arrow A) because, if the sheath is moved in this direction, the frictional engagement between the sheath and the fingers 27 will tend to close the openings 26, 28 and thereby make the removal of the sheath in this direction harder. The fingers also prevent the sheath from being turned within the holes 26, 28.

The openings 26, 28 are not aligned with each other, i.e. the axes 29 of the two openings are out of alignment and form an angle α of 170 to 110°, and so the insertion of the sheath 16 into the opening 26, 28, causes the top element 22 to flex about the hinge 24, which brings the openings closer into alignment. The resilience of the hinge 24 causes the fingers 27 to press against the sheath, which holds the sheath tightly in the openings. When an attempt is made to move the sheath 16 in the direction of the arrow B, the tight engagement of the sheath in the opening 28 causes the upper element to move away from the lower element about the hinge 24, but this brings the two elements out of alignment which makes their engagement in the openings tighter, thereby resisting the sheath from being moved in the direction of arrow B.

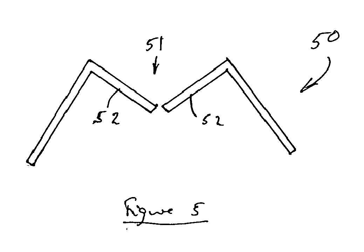

Generally, it has been found that two or more elements will be required to hold the sheath in place but if the force exerted by the fingers 27 is large, then it may be possible to use only one element. However, the use of only one element is assisted if the element is domed, as shown in Figure 4. The element 30 in Figure 4(a) is domed; the curvature may be in only one plane i.e. the element 30 may form parts of the wall of the cylinder, or the curvature may be applied in two directions, e.g. the element 30 may be a section of a dome. A cross shape 32 (as shown in Figure 3(c)) is applied in the element 30 to form the opening 31 and the sheath 16 may be pushed from the concave side of the dome through the opening 31 in the direction of the arrow A, thereby deforming fingers 32 of the opening. As described above, the fingers 32 will resist the movement of the sheath in a direction opposite to that of insertion, i.e. it resists motion in the direction of the arrow B. The dome shape of the element 30 adds extra strength to the element and prevents it from distorting when an attempt is made to pull the sheath 16 in the direction of the arrow B. The additional resistance to the removal of the sheath brought about by the rounded or angled shape of the element 30 means that it is more feasible to use only one element in the present arrangement, in comparison to the two elements provided in the arrangement of Figure 4.

Alternatively, the element shape could be 'M' shaped, as shown for element 40 in Figure 5. In Figure 5, the element 50 has a central 'V' shape with a hole 51 in the bottom such that the side walls 52 act as fingers and bend in to allow insertion of the sheath but the V will prevent retraction of the sheath as the side walls are forced out further as the bottom of the V is lifted up.

Any number of fingers 27, 33 may be provided in the openings 26, 28, 31 and 51 although preferably 2 to 6 fingers are provided. However, 4 fingers are preferred since they fit in with the 4 ridges 17 on the sheath, and thus prevent the sheath from rotating in the opening; if there were not the same number of slots as ridges, the grip strength would be reduced.

The use of the fingers 27, 32 allows for a wide variety of different diameter sheaths to be opened using the arrangement of the present invention.

More than two elements 20, 22 may be provided each connected to the preceding element by a resilient spring 24.

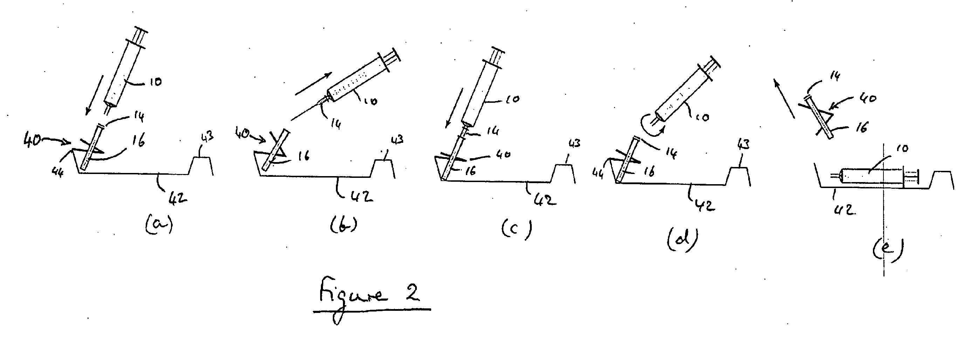

Turning to Figure 2, there is shown, in cross section, a tray 42 which may be made out of shaped paper-pulp or any other suitable material for forming hospital trays.

A device 40 in accordance with the present invention is attached along one edge of the tray 42. The device 40 and the tray 42 may be formed integrally with each other, in which case the addition of the device 40 increases the manufacturing cost of the tray only very slightly. The device is preferably joined to the tray by a line of weakness 44. Alternatively, the device could be made separately and removably attached to the tray 42, e.g. by means of a 'slot fit'.

The syringe 10 and the sheathed needle 14, 16 are provided as separate items in the tray. The needle in its sheath is pushed into the device of the present invention, as described in connection with Figure 3, which holds it in position. The syringe 10 is then pushed onto the syringe outlet 11 such that the collar 13 is held on the outlet 11 by friction.

By either holding the tray 42 in one hand and pulling on the syringe 10 with the other hand, or resting the tray on a surface and pushing the wrist of one hand on the wrist rest 43 and pulling the syringe with the same hand, a medical practitioner can unsheathe the needle 14 (see Figure (b)), which can be put into operation in the normal manner. The use of the wrist rest 43 holds the tray prevents the tray from moving or tipping and has the advantage that the whole procedure can be performed with only one hand leaving the other hand free. Because the user's hand is nowhere near the needle when the syringe is pushed onto the needle, needle stick injury can be avoided if the needle accidentally becomes unsheathed.

After completion of its normal operation, the needle 14 is re-sheathed by pushing it into the sheath 16 (see Figure 2(c)). In this operation, the sheath 16 can engage at the bottom edge of the tray to prevent it being pushed through the device 40. It is important to note that the user can hold the tray with one hand in a position well away from the sheath and the needle 14 and accordingly the risk of needle stick injury in these circumstances is vastly reduced. Alternatively, the user can press down on the wrist rest 43 of the tray with the wrist of the hand which is holding the needle; and insert the needle into the sheath. The tray has been designed to have a wide-stable base, with extra support provided by the wrist support 43 at the back. As such, the operation can be comfortably completed using only one hand. This is the safest method, as it eliminates the possibility of stabbing the other hand which can be kept well away, or the other hand can be used to do another operation such as comfort the patient.

This method further reduces the risk of needle stick injury as the contaminated needle is 'made safe' immediately after use by sheathing it. Currently because of the discouragement of, or ban on, the practice of resheathing needles manually, the exposed needles are kept on their bulky cumbersome syringes which rest in the shallow trays, and are carried across the ward to the sharps bin where they are finally separated from the syringe and disposed of. Often the syringe and the needle will wrongly be placed into the sharps bin together. This practice is costly and dangerous, as the bins fill up quickly and can easily become overfilled with exposed needles poking out through the opening. Many sharps injuries occur at the bin for this reason. This method would eliminate that, as not only is the needle disposed of separately from the syringe, but also the needle is disposed within its sheath, making it double safe.

Because the sheath 16 is held and prevented from rotating in the device 40 of the present invention, the syringe 10 can be removed from the needle by a simple twisting action (see Figure 2(d)) leaving the sheathed needle 14, 16 held by the device 40 while the syringe 10 has been separated and can be placed in the tray 42. The sheathed needle can be removed manually from the device 40 or, alternatively, the device 40 may be removed along the line of weakness 44 and disposed of together with the sheathed needle held by the device. The advantage of removing the device 40 and the sheathed needle is that it can be disposed of in the bin designated for sharp articles, while allowing the tray 42 and the syringe to be disposed of more cheaply and appropriately.

The wrist wrest 43 as described earlier can be folded over into the tray 40 and act as a thumb guard when carrying the tray, thereby preventing the thumb from coming into contact with the contents of the tray. This will further protect the health worker from potential injuries and contamination from other hazardous materials and equipment which may be in the tray. The wrist support may be elongated so that it is approximately the same length as the tray (not shown) so that it is able to be folded over and cover the well of the tray and prevent the contents spilling out; a latch may be provided to keep the folded over cover in place.

The tray used in connection with the present invention may be deeper than the standard trays on the market, making them better designed for hospital ward use. The wrist support 43 has a wide base which provides extra stability. The shape and design of the tray is non intrusive and pleasing to the eye, further improving on the fact that this is also a patient friendly product. The tray can be designed to be stackable.

## Claims

1. A device that is configured to grip the sheath of a medical needle so that the needle can be retracted from it with one hand and to hold the sheath while the needle is in use, which device comprises at least two elements that are resiliently connected together and at least partially define at least one opening into which the sheath can be pushed, said at least two elements and at least one opening acting as a valve that allows the sheath to be pushed into the at least one opening but grips the sheath if the sheath is pulled out of the opening, wherein the resilient connection between the elements is configured to hold a sheath against rotating within the opening.

2. A device as claimed in claim 1, wherein said at least two elements and the at least one opening that together act as a valve comprises a first element having a first opening therein and a second element joined to the first element by a resilient hinge and having a second opening therein, wherein the first and second elements are out of alignment with each other so that, when a sheath is pushed into the first and second openings and engages the first opening, the first element flexes relative to the second element about the resilient hinge and when the sheath is no longer pushed, the resilience in the hinge causes the first and second openings to grip on the sheath.

3. A device as claimed in claim 2, wherein notional lines passing orthogonally through the centres of the first and second openings lie an angle α of 170° to 110° to each other.

4. A device as claimed in claim 2 or claim 3, wherein, when the hinge is unflexed, the first element lies at an angle of 10° to 60° to the second element.

5. A device as claimed in any of claims 2 to 4, which includes at least one further element that has an opening therein and that is resiliently connected to a preceding element by a resilient hinge, and wherein the opening in each further element lies out of alignment with the opening in the preceding element.

6. A device as claimed in claim 1, wherein said at least two elements and the at least one opening that together act as a valve comprises at least two elements resiliently connected together and an opening formed between the elements, wherein the elements are angled with respect to each other in a configuration such that a sheath when moved in one direction through the opening engages the elements bordering the opening and further movement in that direction tends to expand the opening, thereby permitting the sheath to move further in that direction, and such that a sheath when moved in the opposite direction through the opening engages the elements bordering the opening and further movement in that direction tends to contract the opening, thereby resisting the sheath moving in that direction.

7. A device as claimed in claim 6, wherein the elements that are angled with respect to each other from part of a dome or "V" shape.

8. A device as claimed in any preceding claim wherein the elements are integrally formed, e.g. in a single moulding.

9. A holder, e.g. a tray, for holding a medical needle, a sheath fitted over the needle and a syringe, which holder comprises a device as claimed in any one of claims 1 to 8, said device optionally being separable from the rest of the holder, e.g. it is joined to the rest of the holder by a line of weakness, or is a separate piece which is connected to he holder and can removed.

10. A holder, e.g. a tray, for holding a medical needle, a sheath fitted over the needle and a syringe, which holder comprises a device capable of holding the sheath while the needle is removed from inside it, wherein said device is separable from the rest of the holder, e.g. it is joined to the rest of the holder by a line of weakness.

11. A holder as claimed in claim 9 or claim 10, which includes a flat surface on the top of the holder opposite the location of the device that can act as a wrist rest for keeping the holder still while sheathing a needle, which wrist rest can optionally be bent over the holder to provide a thumbguard or a cover for the holder.

12. A method of removing a sheath from a medical needle using a device as claimed in any one of claims 1 to 8, which method comprises:
moving the sheath into the at least one opening in a first direction;
pushing the sheath into the at least one opening to engage the elements bordering the at least one opening;
releasing the pushing force to allow the elements to grip the sheath; and
retracting the medical needle from the gripped sheath, e.g. by pulling the medical needle in the direction opposite to the first direction, the method optionally also including the steps of:
reinserting the needle into the sheath held by the device, and
twisting and pulling a syringe connected to the needle to disconnect the syringe from the needle.

13. A system for preventing needle stick, the system comprising a needle held within a sheath and a flexible wrapper, the inner surface of the wrapper including a first area of adhesive that adheres the sheath to the wrapper and a second area of contact adhesive that is accessible when the wrapper is open and that can be used to adhere the wrapper to a surface to immobilise the wrapper.
